# EUROPÄISCHE PATENTSCHRIFT

(11) **EP 0 540 774 B1**
(45) Veröffentlichungstag und Bekanntmachung des Hinweises auf die Patenterteilung: **03.05.1995**
(21) Anmeldenummer: 91118993.4
(22) Anmeldetag: 07.11.1991
(51) Int. Cl.: B05B 7/00, A61M 11/06

(54) **Flüssigkeitszerstäubervorrichtung**
Fluid atomizing device
Dispositif de pulvérisation de liquide

(43) Veröffentlichungstag der Anmeldung: 12.05.1993
(73) Patentinhaber: PAUL RITZAU PARI-WERK GmbH, D-82319 Starnberg (DE)
(72) Erfinder: Knoch, Martin, Dr.Ing., W-8137 Berg (DE); Lintl, Andreas, W-8130 Starnberg (DE)
(74) Vertreter: Eitle, Werner, Dipl.-Ing.

(56) Entgegenhaltungen:
- EP-A- 0 171 726
- DE-U- 8 437 274
- DE-U- 8 614 551
- DE-U- 8 905 364
- GB-A- 2 023 023
- PATENT ABSTRACTS OF JAPAN vol. 13, no. 293 (C-615)6. Juli 1989 & JP-1 086 982

## Beschreibung

Die Erfindung betrifft eine Flüssigkeitszerstäubervorrichtung zur Erzeugung eines Aerosols inbesondere für Inhalationszwecke.

Flüssigkeitszerstäuber für Inhalationszwecke sind in verschiedenartigen Ausgestaltungen bekannt. So ist beispielsweise aus DE 32 38 149 A1 eine Zerstäubervorrichtung bekannt, die aus einem unteren und einem oberen Gehäuseteil besteht. Der untere Gehäuseteil bildet den Behälter für die zu zerstäubende Flüssigkeit und weist einen mit einer Druckgaszuleitung ausgestatteten Sockel auf. Im Inneren des Gehäuses ist davon getrennt ein Düsenkörper in Fortsetzung des Druckgaskanals und einem im oberen Gehäuseteil ausgebildeten Luftzufuhrkamin gegenüberliegend vorgesehen. Der Düsenkörper ist am unteren Gehäuseteil lösbar befestigt. Zwischen Düsenkörper und Luftzufuhrkamin ist wiederum getrennt von den übrigen Bestandteilen ein Gasstromsteuer angeordnet, das zum Ablenken und Verteilen der zerstäubten Flüssigkeit dient, die aus dem dem Luftzufuhrkanal zugewandten Ende des Düsenkörpers austritt. In dem oberen Gehäuseteil ist seitlich ein Ausströmstutzen für das Aerosol angeformt. Der Düsenkörper weist zwei Bohrungen auf, durch die die Flüssigkeit aus einem Flüssigkeitssammelbereich des unteren Gehäuseteils angesaugt wird. Um eine Reinigung der Düsenkanäle sowie der gesamten Zerstäubervorrichtung durchführen zu können, ist der untere Gehäuseteil vom oberen Gehäuseteil bzw. der Düsenkörper vom unteren Gehäuseteil trennbar.

Aus DE-C-34 29 389 ist eine andere Flüssigkeitszerstäubervorrichtung bekannt, die aus einem oberen Gehäuseteil mit Luftzufuhrkanal und einem unteren Gehäuseteil mit Druckgasanschluß besteht. Am oberen Gehäuseteil ist seitlich ein Auslaßstutzen für das im Gehäuseinneren erzeugte Aerosol und im oberen Bereich mittig ein in das Gehäuseinnere ragender Luftzufuhrkamin angeformt. An dem im unteren Gehäuseteil mittig vorgesehenen Druckgasanschluß ist lösbar ein Düsenkörper angebracht, der am unteren Ende den Anschlußteil eines zentrisch verlaufenden Druckgaskanals aufweist. Der Düsenkörper ist fluchtend zu dem Luftzufuhrkamin angeordnet, so daß das Zerstäubungsende des Düsenkörpers dem Luftzufuhrkamin gegenüberliegt. Am Zerstäubungsende des Düsenkörpers ist eine Aufnahme für einen weiteren Bestandteil der Zerstäubervorrichtung vorgesehen, die ein Gasstromsteuer trägt. Dieser Bestandteil wird auf den Düsenkörper so aufgesetzt, daß das Gasstromsteuer oberhalb des Zerstäubungsendes den Öffnungen des Druckgaskanals und den Flüssigkeitskanälen gegenüberliegend angeordnet ist. Ferner sind bei dem bekannten Zerstäuber zwei ringförmige Umlenkelemente vorgesehen, die im Inneren des Gehäuses entlang der Gehäusewandung angeordnet sind, so daß eine Umlenkung und Verteilung, sowie eine Beruhigung des erzeugten Aerosols bewirkt wird.

Die beiden beispielhaft beschriebenen Zerstäuber weisen einen Nachteil auf, der auch den anderen bisher bekannten Zerstäubern anhaftet. Da nämlich die Zerstäuber, insbesondere die Düsenkörper, stets sorgfältig gereinigt werden müssen, um Rückstände von Aerosol-Flüssigkeiten vollständig zu entfernen, müssen auch die bisher bekannten Zerstäuber derart zerlegbar sein, daß eine Reinigung des Düsenkörpers und insbesondere der Flüssigkeitskanäle möglich ist. Dazu sind die bisher bekannten Zerstäuber in mehrere, oftmals sehr kleine Teile zerlegbar. Der Benutzer muß daher nicht nur die Reinigung sehr sorgfältig durchführen, sondern muß in der Lage sein, die Zerstäubervorrichtung in ihre einzelnen Komponenten zu zerlegen und wieder zusammensetzen zu können. Dabei besteht auch die nicht unerhebliche Gefahr, daß eines der kleinen Teile der Zerstäubervorrichtung verloren geht.

Jedoch ist es erforderlich, daß die Ausbildung des Düsenkörpers, insbesondere der Flüssigkeitskanäle sowie die Lage des Gasstromsteuers in Bezug auf das Zerstäubungsende des Düsenkörpers innerhalb geringer Toleranzen liegt. Denn die die Strömung des Flüssigkeit/Luft-Gemisches beeinflußenden Bestandteile müssen zur Erzeugung eines feinverteilten und gut durchmischten, sowie homogenen Aerosols in genauen Abständen und Beziehungen zueinander angeordnet sein. Aus diesem Grund wurde bislang der Düsenkörper getrennt vom Gehäuse des Zerstäubers angefertigt und stets getrennt darin eingesetzt. In den meisten Fällen wurden auch die weiteren, die Strömung beeinflußenden Teile getrennt vom Gehäuse hergestellt und lösbar mit diesem verbunden.

Aus GB-A-2 023 023, das dem Oberbegriff des Anspruchs 1 entspricht, ist eine Flüssigkeitszerstäubervorrichtung bekannt, die aus insgesamt fünf Einzelteilen besteht. Ein erster, unterer Gehäuseteil bildet nicht nur ein Reservoir für die zu zerstäubende Flüssigkeit, sondern umfaßt auch einen inneren Teil einer Zerstäuberdüse. Ein zweiter Gehäuseteil kann auf den ersten Gehäuseteil aufgesteckt werden und nimmt in seinem Inneren den äußeren Teil der Zerstäuberdüse auf, der auf den inneren Teil der Zerstäuberdüse aufgesteckt wird. Der äußere Teil der Zerstäuberdüse ist einstückig mit einem zylindrischen Element verbunden, das einen Zerstäubungsraum unmittelbar vor der Öffnung der Zerstäuberdüse festlegt. Abgeschlossen wird der Zerstäubungsraum durch eine Kappe, die gleichzeitig zur Halterung des Bauteils, bestehend aus äußerem Zerstäuberdüsenteil und zylindrischem Element dient.

Ausgehend von diesem Stand der Technik liegt der Erfindung die Aufgabe zugrunde, eine Flüssigkeitszerstäubervorrichtung zu schaffen, die aus einer geringeren Anzahl von Einzelteilen besteht und dennoch eine gründliche Reinigung der mit der Aerosol-Flüssigkeit in Berührung kommenden Teilen ermöglicht.

Gelöst wird diese Aufgabe durch eine Zerstäubervorrichtung mit den Merkmalen des Patentanspruchs 1.

Weitere vorteilhafte Ausgestaltungen ergeben sich aus den Unteransprüchen.

Vorzugsweise besteht die Zerstäubervorrichtung nur aus zwei Gehäuseteilen, die miteinander in Eingriff gebracht werden können, so daß sich die vorgeschriebenen Abstände innerhalb des den Düsenkörper bildenden Abschnitts quasi von selbst einstellen. Dazu ist zumindest eines der Gehäuseteile mit einem federnden Element ausgestattet, das aber integral mit dem Gehäuseteil ausgebildet ist. Vorzugsweise handelt es sich dabei um einen geschwächten Wandungsabschnitt.

Für den Benutzer ist der zweiteilige Aufbau der erfindungsgemäßen Zerstäubervorrichtung vorteilhaft und führt dazu, daß auf sehr einfache Weise die Zerstäubervorrichtung zerlegt und gereinigt werden kann. Dies gilt insbesondere im Hinblick auf die Flüssigkeitskanäle, die bei einer vorteilhaften Ausgestaltung nach dem Zerlegen der Zerstäubervorrichtung unmittelbar zuganglich sind.

Die Zerstäubervorrichtung kann aber auch einen äußeren Düsenkörper aufweisen, der vom Gehäuseteil lösbar ist, so daß für den Düsenkörper etwa ein anderes Material oder eine andere Herstellungsart gewählt werden kann.

Im folgenden wird die Erfindung anhand eines Ausführungsbeispiels und unter Bezugnahme auf die Zeichnungen genauer beschrieben, in denen zeigt:
- Fig. 1: eine geschnittene Ansicht eines ersten Gehäuseteils einer erfindungsgemäßen Flüssigkeitszerstäubervorrichtung,
- Fig. 2: eine teilweise geschnittene Ansicht eines zweiten Gehäuseteils einer erfindungsgemäßen Flüssigkeitszerstäubervorrichtung,
- Fig. 3: eine vergrößerte, teilweise geschnittene Darstellung eines Bereichs des zweiten Gehäuseteils aus Fig. 2, und
- Fig. 4: die aus den beiden Gehäuseteilen aus Fig. 1 und 2 zusamengesetzte erfindungsgemäße Flüssigkeitszerstäubervorrichtung in geschnittener Darstellung.

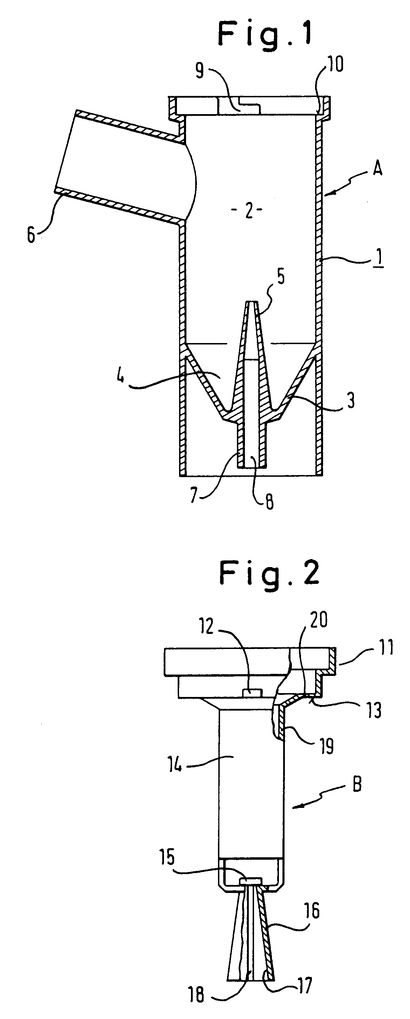

In Fig. 1 ist ein erster Gehäuseteil A eines Ausführungsbeispiels der erfindungsgemäßen Flüssigkeitszerstäubervorrichtung dargestellt. Der zylindrische Grundkörper 1 umschließt einen Vernebelungsraum 2, der nach unten durch eine Wandung 3 eines Flüssigkeitssammelbereichs 4 und eines inneren Teils 5 der Zerstäuberdüse abgeschlossen ist. Seitlich ist an dem ersten Gehäuseteil A ein Aerosol-Auslaßstutzen 6 angeordnet. Der zylindrische Grundkörper 1, die Wandung 3 des Flüssigkeitssammelraums, der innere Teil 5 der Zerstäuberdüse und der Aerosol-Anschlußstutzen 6 sind einstückig ausgebildet.

Der innere Teil 5 der Zerstäuberdüse ist vorzugsweise spitzkegelig ausgestaltet und weist am außerhalb des Vernebelungsraums 2 liegenden Ende einen Anschluß 7 für die Druckgasleitung auf, über die das unter Druck stehende Gas, zumeist Luft, einem Druckgaskanal 8 im inneren Teil 5 der Zerstäuberdüse zugeführt wird. An dem in den Vernebelungsraum 2 ragenden Ende des inneren Teils 5 tritt das zugeführte Druckgas aus.

An dem dem Flüssigkeitssammelbereich 4 gegenüberliegenden Ende weist der erste Gehäuseteil eine erste Verbindungseinrichtung 9, vorteilhaft in Form von Nuten eines Bajonettverschlusses auf. Als Verbindungseinrichtung 9 kann auch ein geeignetes Gewinde oder ein Schnappverschluß vorgesehen werden. Ferner ist am ersten Gehäuseteil A eine erste Anschlagfläche 10 vorgesehen, die mit dem im folgenden beschriebenen zweiten Gehäuseteil zusammenwirkt.

In Fig. 2 ist der zweite Gehäuseteil B des Ausführungsbeispiels der erfindungsgemäßen Zerstäubervorrichtung dargestellt. Auch der zweite Gehäuseteil B ist im wesentlichen rotationssymmetrisch. An seinem oberen Ende weist der zweite Gehäuseteil B eine zweite Verbindungseinrichtung 11 auf, die zur Verbindungseinrichtung 9 des ersten Gehäuseteils A korrespondiert. In Fig. 2 ist beispielhaft eine Lasche 12 dargestellt, die in die Nuten des in Fig. 1 gezeigten Bajonettverschlusses einrastbar und verriegelbar ist. Eine zweite Anschlagfläche 13 des zweiten Gehäuseteils B ist für den Eingriff mit der Anschlagfläche 10 des ersten Gehäuseteils A vorgesehen.

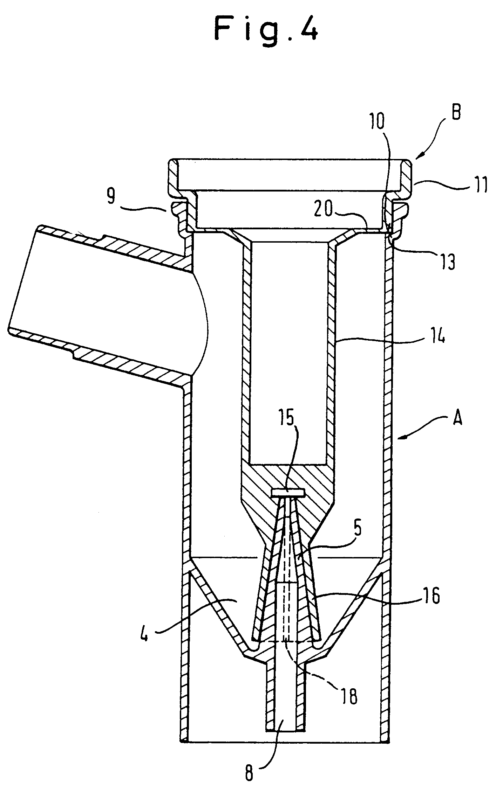

Einstückig mit der zweiten Verbindungseinrichtung 11 ist ein Zuluftkamin 14 ausgebildet, der sowohl an seinem oberen Ende im Bereich der zweiten Verbindungseinrichtung 11 als auch an seinem gegenüberliegenden unteren Ende offen ist. Am unteren Ende des Zuluftkamins 14 ist ferner ein Gasstromsteuer ausgebildet, das quer zur im wesentlichen kreisförmigen Öffnung des Zuluftkamins 14 verläuft. Unterhalb des Gasstromsteuers 15 befindet sich ein äußerer Teil 16 der Zerstäuberdüse, der, wie weiter unten im Zusammenhang mit Fig. 4 noch genauer beschrieben wird, mit dem inneren Teil 5 der Zerstäuberdüse zusammengefügt wird. Die innere Wand 17 des äußeren Teils 16 der Zerstäuberdüse ist entsprechend der Form des inneren Teils 5 der Zerstäuberdüse ausgebildet. Ferner weist die innere Wand zumindest eine, vorzugsweise aber zwei diametral gegenüberliegende Nuten 18 auf, die die Flüssigkeitskanäle bilden. Werden zwei Nuten 18 vorgesehen, sind sie vorzugsweise beidseitig vom Gasstromsteuer 15 so anzuordnen, daß eine Verbindungsgerade zwischen ihnen senkrecht zur Ebene des Gasstromsteuers 15 verläuft.

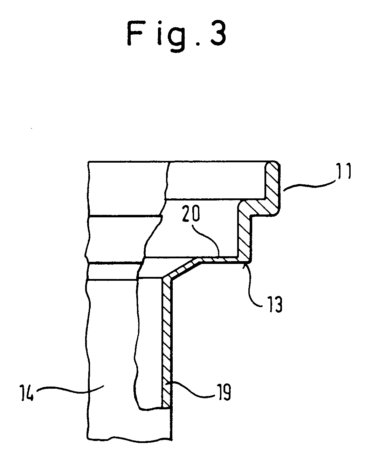

In Fig. 3 ist der obere Bereich des zweiten Gehäuseteils B in vergrößerter Darstellung gezeigt. In dieser Darstellung ist die Verbindungseinrichtung des zweiten Gehäuseteils B sowie die Anschlagfläche 13 deutlich erkennbar. Ferner ist die Wandung 19 des Zuluftkamins 14 in geschnittener Darstellung gezeigt. Deutlich erkennbar ist auch, daß der Wandungsteil 20, der die zweite Verbindungseinrichtung 11 mit dem Zuluftkamin 14 verbindet, eine geringere Wandstärke aufweist als die Wandung 19 des Zuluftkamins bzw. die zweite Verbindungeinrichtung 11. Aufgrund dieser Ausgestaltung kann, wie im folgenden unter Bezugnahme auf Fig. 4 noch ausführlich beschrieben wird, der Zuluftkamin 14 und mit ihm der äußere Teil (16) der Zerstäuberdüse bezogen auf die zweite Verbindungseinrichtung 11 des zweiten Gehäuseteils B verschoben werden. Denn der Wandbereich 20 wirkt als federndes Element, das die Verbindung zwischen dem Zuluftkamin 14 und der zweiten Verbindungseinrichtung 11 herstellt. Alternativ kann auch ein Faltenbalg vorgesehen werden, der eine Verschiebung zwischen Zuluftkamin 14 und der zweiten Verbindungseinrichtung 11 des zweiten Gehäuseteils B erlaubt.

In Fig. 4 ist die erfindungsgemäße Zerstäubervorrichtung in zusammengesetztem Zustand dargestellt.

Der äußere Teil 16 der Zerstäuberdüse sitzt auf dem inneren Teil 5 der Zerstäuberdüse auf, da die Aussen- bzw. Innenfläche der beiden Teile entsprechend aneinander angepaßt sind. Die äußere Wand des inneren Teils 5 bildet zusammen mit den Nuten 18, die in der inneren Wand 17 des äusseren Teils 16 vorgesehen sind, Flüssigkeitskanäle, die den Flüssigkeitssammelbereich mit dem oberen Ende der Zerstäuberdüse verbinden. Eine geeignete Materialwahl sowie die spitzkegelige Ausgestaltung der beiden in Eingriff stehenden Flächen des inneren bzw. äusseren Teils der Zerstäuberdüse sorgen für eine exakte Ausrichtung der Flüssigkeitskanäle 18 und des Druckgaskanals 8, insbesondere im oberen Bereich, d.h. in Bezug auf das Gasstromsteuer 15. Die Längentoleranzen, die insbesondere in diesem Bereich einzuhalten sind, werden aufgrund der selbständig eintretenden Ausrichtung der miteinander in Eingriff stehenden Teile 5 und 16 eingehalten. Eine entsprechende Ausgestaltung des spitzkegligen inneren Teils 5 sowie des äusseren Teils 16 der Zerstäuberdüse wirft keine besonderen fertigungstechnischen Probleme auf, so daß auch eine Massenproduktion unter Einhaltung der engen Toleranzen in Bezug auf die die Strömung des Flüssigkeit/Luft-Gemisches beeinflussenden Teile kostengünstig möglich ist. Denn die hier Einfluß ausübenden Bestandteile sind dicht beieinander angeordnet und können gut aufeinander ausgerichtet werden.

Im Bereich der Verbindungseinrichtung 9 und 11 des ersten bzw. zweiten Gehäuseteils liegt die Anschlagfläche 10 des ersten Gehäuseteils A an der Anschlagfläche 13 des zweiten Gehäuseteils B an. Mit Hilfe der vorgesehenen Verbindungseinrichtungen ist der erste Gehäuseteil A mit dem zweiten Gehäuseteil B fest, aber lösbar verbunden. Obwohl die Toleranzanforderungen in diesem Bereich des Zerstäubers nicht so hoch sind, können auch die Verbindungseinrichtungen mit engen Toleranzen ausgebildet werden, so daß eine fest sitzende Verriegelung und eine gute Abdichtung des Verneblungsraumes gewährleistet ist. Denn der Wandbereich 20 ermöglicht eine Ausgleichsverschiebung in axialer Richtung, so daß die Einhaltung von engen Toleranzen zwischen dem Bereich der beiden Verbindungseinrichtungen 9 bzw. 11 und dem Bereich der Zerstäuberdüse nicht erforderlich ist.

Einzige Voraussetzung ist, daß der Zuluftkamin 14 ausreichend lang ist, um einen festen Sitz des äusseren Teils 16 der Zerstäuberdüse auf dem inneren Teil 5 zu gewährleisten, wobei gleichzeitig die Anschlagfläche 10 in Eingriff mit der Anschlagfläche 13 stehen muß. Die Wandung 20 ist dann im zusammengesetzten Zustand der Zerstäubervorrichtung leicht nach oben durchgebogen und übt eine in Richtung auf einen festen Sitz wirkende Kraft auf die zusammengesetzte Zerstäuberdüse aus, wodurch in diesem wichtigen Bereich eine exakte Ausrichtung und Einhaltung der vorgegebenen Toleranzen erreicht wird.

Bei dem zuvor geschilderten Ausführungsbeispiel wirkte der Wandbereich 20 als federndes Element und gestattete eine Verschiebung des Zuluftkamins in Längsrichtung der Zerstäubervorrichtung. Auf ähnliche Weise kann aber auch ein federndes Element in Form eines Faltenbalgs im oberen Bereich des Zuluftkamins 14 vorgesehen werden. Auch am ersten Gehäuseteil bieten sich Möglichkeiten, einen federnden Bereich vorzusehen, etwa im Bereich der Wandung 3 des Flüssigkeitssammelraums 4. Federnde Elemente können aber auch an beiden Gehäuseteilen vorgesehen werden. Auf diese Weise ist eine axiale Verschiebung des Bereichs der Zerstäuberdüse zu dem Bereich der Verbindungseinrichtungen möglich, so daß sich eine exakte Ausrichtung der den Düsenkörper bildenden Teile einstellt, ohne daß Toleranzen über eine lange Strecke hinweg, nämlich zwischen den beiden genannten Bereichen eingehalten werden müssen. Der als federndes Element wirkende Wandbereich sollte im Hinblick auf eine Verdrehung ausreichend steif sein, um eine verdrehte Positionierung der Flüssigkeitskanäle 18 in Bezug auf den Auslaßstutzen 6 zu vermeiden. Bei dem im Ausführungsbeispiel gezeigten Wandbereich 20 ist dies ohne weiteres gegeben. Jedoch sind Ausführungsformen möglich, die eine Beachtung dieses Aspekts verlangen.

## Patentansprüche

1. Flüssigkeitszerstäubervorrichtung mit
- einem ersten Gehäuseteil (A), der
-- einen inneren Teil (5) einer Zerstäuberdüse und
-- eine erste Verbindungseinrichtung (9) für die Verbindung des ersten Gehäuseteils mit einem weiteren Gehäuseteil zur Bildung eines Vernebelungsraumes (2)
umfaßt, und
- einem zweiten Gehäuseteil (B), der
-- eine zweite Verbindungseinrichtung (11), die für die Verbindung des zweiten Gehäuseteils mit dem ersten Gehäuseteil mit der ersten Verbindungseinrichtung (9) in Eingriff bringbar ist,
umfaßt,
dadurch gekennzeichnet, daß
- der zweite Gehäuseteil (B) einen äußeren Teil (16) der Zerstäuberdüse, der mit dem inneren Teil (5) zur Bildung der Zerstäuberdüse in Eingriff bringbar ist, umfaßt und
- ein federndes Element (20) vorgesehen ist, das zumindest an dem ersten oder dem zweiten Gehäuseteil derart angeordnet ist, daß eine Ausgleichsverschiebung zwischen den in Eingriff gebrachten Teilen (5, 16) der Zerstäuberdüse und den in Eingriff gebrachten Verbindungseinrichtungen (9, 11) zuläßt.

2. Flüssigkeitszerstäubervorrichtung nach Anspruch 1,
dadurch **gekennzeichnet**, daß das federnde Element (20) einstückig mit dem Gehäuseteil (A,B) ausgebildet ist.

3. Flüssigkeitszerstäubervorrichtung nach Anspruch 2,
dadurch **gekennzeichnet**, daß das federnde Element (20) ein nachgiebiger Wandbereich des Gehäuseteils (A, B) ist.

4. Flüssigkeitszerstäubervorrichtung nach einem der Ansprüche 1 bis 3,
dadurch **gekennzeichnet**, daß der innere Teil (5) einstückig mit dem ersten Gehäuseteil (A) ausgebildet ist.

5. Flüssigkeitszerstäubervorrichtung nach einem der Ansprüche 1 bis 4,
dadurch **gekennzeichnet**, daß der innere Teil (5) spitzkeglig mit einer äusseren Wand für den Eingriff mit dem äusseren Teil (16) ausgebildet ist.

6. Flüssigkeitszerstäubervorrichtung nach einem der Ansprüche 1 bis 5,
dadurch **gekennzeichnet**, daß der innere Teil (5) im Inneren einen Druckluftkanal (8) aufweist.

7. Flüssigkeitszerstäubervorrichtung nach einem der Ansprüche 1 bis 6,
dadurch **gekennzeichnet**, daß der innere Teil (5) einstückig mit einer Wandung (3) eines Flüssigkeitssammelbereichs (4) im ersten Gehäuseteil (A) ausgebildet ist.

8. Flüssigkeitszerstäubervorrichtung nach einem der Ansprüche 1 bis 7,
dadurch **gekennzeichnet**, daß der innere Teil (5) einen Druckgasanschluß (7) fortsetzend ausgebildet ist.

9. Flüssigkeitszertäubervorrichtung nach einem der Ansprüche 1 bis 8,
dadurch **gekennzeichnet**, daß die erste Verbindungseinrichtung (9) eine Anschlagfläche (10) aufweist.

10. Flüssigkeitszerstäubervorrichtung nach einem der Ansprüche 1 bis 9,
dadurch **gekennzeichnet**, daß die erste Verbindungseinrichtung (9) Einsteck- und Führungsschlitze eines Bajonettverschlusses aufweist.

11. Flüssigkeitszerstäubervorrichtung nach einem der Ansprüche 1 bis 10,
dadurch **gekennzeichnet**, daß der äussere Teil (16) einstückig mit dem zweiten Gehäuseteil (B) ausgebildet ist.

12. Flüssigkeitszerstäubervorrichtung nach einem der Ansprüche 1 bis 11,
dadurch **gekennzeichnet**, daß der äussere Teil (16) innen spitzkeglig mit einer inneren Wand (17) ausgebildet ist.

13. Flüssigkeitszerstäubervorrichtung nach Anspruch 12,
dadurch **gekennzeichnet**, daß die innere Wand (17) des äusseren Teils (16) zumindest eine Nut (18) zur Bildung zumindest eines Flüssigkeitskanals aufweist.

14. Flüssigkeitszerstäubervorrichtung nach einem der Ansprüche 1 bis 13,
dadurch **gekennzeichnet**, daß der zweite Gehäuseteil (B) ein Gasstromsteuer (15) aufweist, das dem Austrittsende der Zerstäuberdüse gegenüberliegend angeordnet ist.

15. Flüssigkeitszerstäubervorrichtung nach einem der Ansprüche 1 bis 14,
dadurch **gekennzeichnet**, daß der zweite Gehäuseteil (B) einen Zuluftkamin (14) umfaßt, an dessen einem Ende die zweite Verbindungseinrichtung (11) und an dessen anderem Ende der äussere Teil (16) der Zerstäuberdüse angeordnet ist.

16. Flüssigkeitszerstäubervorrichtung nach einem der Ansprüche 1 bis 15,
dadurch **gekennzeichnet**, daß der zweite Gehäuseteil (B) eine Anschlagfläche (13) aufweist.

17. Flüssigkeitszerstäubervorrichtung nach einem der Ansprüche 1 bis 16,
dadurch **gekennzeichnet**, daß die zweite Verbindungseinrichtung (11) die Laschen (12) eines Bajonettverschlusses umfaßt.

18. Flüssigkeitszerstäubervorrichtung nach einem der Ansprüche 1 bis 17,
dadurch **gekennzeichnet**, daß die erste und die zweite Verbindungseinrichtung (9, 11) ein Gewinde oder eine Schnappvebindung ist.

19. Flüssigkeitszerstäubervorrichtung nach einem der Ansprüche 1 bis 18,
dadurch **gekennzeichnet**, daß an dem ersten Gehäuseteil (A) ein Aerosolauslaßstutzen (6) angeformt ist.

## Claims

1. Liquid atomiser device with
- a first housing portion (A) which includes
- an inner portion (5) of an atomiser nozzle and
- a first connecting device (9) for connection of the first housing portion to a further housing portion to form an atomisation chamber (2), and
- a second housing portion (B) which includes
- a second connecting device (11) which can be brought into engagement with the first connecting device (9) for connection of the second housing portion to the first housing portion,
characterised in that
- the second housing portion (B) includes an outer portion (16) of the atomiser nozzle which can be brought into engagement with the inner portion (5) to form the atomiser nozzle and
- a spring element (20) is provided which is arranged at least on the first or second housing portion in such a way that it allows compensatory displacement between the engaged portions (5, 16) of the atomiser nozzle and the engaged connecting devices (9, 11).

2. Liquid atomiser device according to claim 1, characterised in that the spring element (20) is constructed in one piece with the housing portion (A, B).

3. Liquid atomiser device according to claim 2, characterised in that the spring element (20) is a flexible wall region of the housing portion (A, B).

4. Liquid atomiser device according to any of claims 1 to 3, characterised in that the inner portion (5) is constructed in one piece with the first housing portion (A).

5. Liquid atomiser device according to any of claims 1 to 4, characterised in that the inner portion (5) is of pointed conical construction with an outer wall for engagement with the outer portion (16).

6. Liquid atomiser device according to any of claims 1 to 5, characterised in that the inner portion (5) comprises in the interior a compressed air channel (8).

7. Liquid atomiser device according to any of claims 1 to 6, characterised in that the inner portion (5) is constructed in one piece with a wall (3) of a liquid collecting zone (4) in the first housing portion (A).

8. Liquid atomiser device according to any of claims 1 to 7, characterised in that the inner portion (5) is constructed in extension of a compressed gas connection (7).

9. Liquid atomiser device according to any of claims 1 to 8, characterised in that the first connecting device (9) comprises a stop face (10).

10. Liquid atomiser device according to any of claims 1 to 9, characterised in that the first connecting device (9) comprises insertion and guide slots of a bayonet fastening.

11. Liquid atomiser device according to any of claims 1 to 10, characterised in that the outer portion (16) is constructed in one piece with the second housing portion (B).

12. Liquid atomiser device according to any of claims 1 to 11, characterized in that the outer portion (16) on the inside is of pointed conical construction with an inner wall (17).

13. Liquid atomiser device according to claim 12, characterised in that the inner wall (17) of the outer portion (16) comprises at least one groove (18) to form at least one liquid channel.

14. Liquid atomiser device according to any of claims 1 to 13, characterised in that the second housing portion (B) comprises a gas flow control surface (15) which is arranged opposite the outlet end of the atomiser nozzle.

15. Liquid atomiser device according to any of claims 1 to 14, characterised in that the second housing portion (B) includes an air supply flue (14) at one end of which is arranged the second connecting device (11) and at the other end of which is arranged the outer portion (16) of the atomiser nozzle.

16. Liquid atomiser device according to any of claims 1 to 15, characterised in that the second housing portion (B) comprises a stop face (13).

17. Liquid atomiser device according to any of claims 1 to 16, characterised in that the second connecting device (11) includes the strips (12) of a bayonet fastening.

18. Liquid atomiser device according to any of claims 1 to 17, characterised in that the first and second connecting device (9, 11) is a thread or a snap-action connection.

19. Liquid atomiser device according to any of claims 1 to 18, characterized in that an aerosol outlet connection (6) is formed integrally with the first housing portion (A).

## Revendications

1. Dispositif de pulvérisation de liquide
- avec une première partie de boitier (A) comprenant
-- une partie intérieure (5) d'une buse de pulvérisation et
-- un premier dispositif d'assemblage (9) pour la liaison de la première partie de boîtier avec une autre partie de boîtier en vue de la formation d'une chambre de
nébulisation (2), et
- avec une deuxième partie de boîtier (B) comprenant
-- un deuxième dispositif d'assemblage (11) qui peut être amené en prise avec le premier dispositif d'assemblage (9) en vue de la liaison de la deuxième partie de boîtier avec la première partie de boîtier,
caractérisé par le fait que
- la deuxième partie de boîtier (B) comprend une partie extérieure (16) de la buse de pulvérisation, qui peut être amenée en prise avec la partie intérieure (5) en vue de la formation de la buse de pulvérisation, et
- il est prévu un élément élastique (20) qui est disposé au moins sur la première partie de boîtier ou la deuxième partie de boîtier de telle manière qu'il permette un mouvement de translation de compensation entre les parties (5, 16) de la buse de pulvérisation, amenées en prise, et les dispositifs d'assemblage (9, 11) amenés en prise.

2. Dispositif de pulvérisation de liquide suivant revendication 1, caractérisé par le fait que l'élément élastique (20) est réalisé d'une seule pièce avec la partie de boîtier (A, B).

3. Dispositif de pulvérisation de liquide suivant revendication 2, caractérisé par le fait que l'élément élastique (20) est une zone de paroi souple de la partie de boîtier (A, B).

4. Dispositif de pulvérisation de liquide suivant l'une des revendications 1 à 3, caractérisé par le fait que la partie intérieure (5) est réalisée d'une seule pièce avec la première partie de boîtier (A).

5. Dispositif de pulvérisation de liquide suivant l'une des revendications 1 à 4, caractérisé par le fait que la partie intérieure (5) est réalisée sous forme de cône pointu avec une paroi extérieure susceptible d'entrer en prise avec la partie extérieure (16).

6. Dispositif de pulvérisation de liquide suivant l'une des revendications 1 à 5, caractérisé par le fait que la partie intérieure (5) présente intérieurement un canal d'air sous pression (8).

7. Dispositif de pulvérisation de liquide suivant l'une des revendications 1 à 6, caractérisé par le fait que la partie intérieure (5) est réalisée d'une seule pièce avec une paroi (3) d'une zone collectrice de liquide (4) dans la première partie de boîtier (A).

8. Dispositif de pulvérisation de liquide suivant l'une des revendications 1 à 7, caractérisé par le fait que la partie intérieure (5) est réalisée de manière à prolonger un raccord de gaz sous pression (7).

9. Dispositif de pulvérisation de liquide suivant l'une des revendications 1 à 8, caractérisé par le fait que le premier dispositif d'assemblage (9) présente une surface de butée (10).

10. Dispositif de pulvérisation de liquide suivant l'une des revendications 1 à 9, caractérisé par le fait que le premier dispositif d'assemblage (9) présente des fentes d'emboîtement et de guidage d'un système à baïonnette.

11. Dispositif de pulvérisation de liquide suivant l'une des revendications 1 à 10, caractérisé par le fait que la partie extérieure (16) est réalisée d'une seule pièce avec la deuxième partie de boîtier (B).

12. Dispositif de pulvérisation de liquide suivant l'une des revendications 1 à 11, caractérisé par le fait que la partie extérieure (16) est réalisée intérieurement sous forme de cône pointu avec une paroi intérieure (17).

13. Dispositif de pulvérisation de liquide suivant la revendication 12, caractérisé par le fait que la paroi intérieure (17) de la partie extérieure (16) présente au moins une rainure (18) en vue de la formation d'au moins un canal de liquide.

14. Dispositif de pulvérisation de liquide suivant l'une des revendications 1 à 13, caractérisé par le fait que la deuxième partie de boitier (B) présente un organe de commande de flux du gaz (15) qui est disposé en face de l'extrémité de sortie de la buse de pulvérisation.

15. Dispositif de pulvérisation de liquide suivant l'une des revendications 1 à 14, caractérisé par le fait que la deuxième partie de boîtier (B) comprend une cheminée d'arrivée d'air (14) à l'une des extrémités de laquelle est disposé le deuxième dispositif d'assemblage (11) et à l'autre extrémité de laquelle est disposée la partie extérieure (16) de la buse de pulvérisation.

16. Dispositif de pulvérisation de liquide suivant l'une des revendications 1 à 15, caractérisé par le fait que la deuxième partie de boîtier (B) présente une surface de butée (13).

17. Dispositif de pulvérisation de liquide suivant l'une des revendications 1 à 16, caractérisé par le fait que le deuxième dispositif d'assemblage (11) comprend des pattes ou oreilles (12) d'un système à baïonnette.

18. Dispositif de pulvérisation de liquide suivant l'une des revendications 1 à 17, caractérisé par le fait que le premier et le deuxième dispositifs d'assemblage (9, 11) comprennent un filetage ou un système à encliquetage.

19. Dispositif de pulvérisation de liquide suivant l'une des revendications 1 à 18, caractérisé par le fait qu'une tubulure de sortie d'aérosol (6) est formée sur la première partie de boîtier (A).
